# EUROPEAN PATENT APPLICATION

(11) **EP 4 542 226 A1**
(43) Date of publication of application: **23.04.2025**
(21) Application number: 23823441.3
(22) Date of filing: 07.02.2023
(51) Int. Cl.: G01N 33/50, C07K 1/30, G01N 27/447, G01N 27/62, G01N 30/06, G01N 30/88, G01N 33/66

(54) **METHOD FOR PREPARING SAMPLE FOR ANALYSIS AND METHOD FOR ANALYZING SAME**

(30) Priority: 14.06.2022 JP 2022095725
(71) Applicant: SHIMADZU CORPORATION, Kyoto-shi, Kyoto 604-8511 (JP)
(72) Inventor: NISHIKAZE, Takashi, Kyoto-shi, Kyoto 604-8511 (JP); FURUKAWA, Jun-ichi, Sapporo-shi, Hokkaido 060-0808 (JP); HANAMATSU, Hisatoshi, Sapporo-shi, Hokkaido 060-0808 (JP)
(74) Representative: Müller-Boré & Partner Patentanwälte PartG mbB
(86) International application number: PCT/JP2023/003906
(87) International publication number: WO 2023/243136

(57) **Abstract**

Provided is a method for analyzing an O-linked sugar chain subjected to linking mode-specific modification of sialic acid, the method including a modification step of adding a modifier that subjects the O-linked sugar chain to linking mode-specific modification of sialic acid, to a sample including a glycoprotein to which the O-linked sugar chain is linked, a releasing step of releasing the O-linked sugar chain subjected to linking mode-specific modification of sialic acid, from the glycoprotein, and an analysis step of analyzing the released O-linked sugar chain subjected to linking mode-specific modification of sialic acid.

## Description

### TECHNICAL FIELD

The present invention relates to a method for preparing a specimen for analysis and a method for analyzing the specimen.

### BACKGROUND ART

Sialic acid is contained also in glycoprotein *in vivo,* and in this case, is present mainly at an end of a sugar chain. Sialic acid is likely to be located outside of a glycoprotein molecule, and therefore likely to involve in recognition from other molecules. The linking modes between sialic acids and the adjacent sugars may be different. For example, there are mainly known α2,3- and α2,6-linking modes with respect to a human N-linked sugar chain, and, in addition to these linking modes, α2,8- and α2,9-linking modes with respect to human O-linked sugar chain and sphingoglycolipid. Since sialic acid is recognized from a different molecule depending on the difference in linking mode and can have a different role, it is important to examine the linking mode of sialic acid.

However, sialic acid has a negative charge and sialic acid is easily decomposed, and therefore it is not easy to examine a sialyl sugar chain containing sialic acid. In addition, there is no change in mass of a sugar chain depending on the difference in the linking mode of sialic acid, and it is thus not possible to distinctively examine the linking mode by mass spectrometry.

In order to distinctively examine the linking mode of sialic acid, a method is proposed in which modification that is linking mode-specific for sialic acid is conducted. In this method, a property that α2,3-sialic acid more easily generates intramolecular dehydration by a dehydration-condensation agent than α2,6-sialic acid is utilized, and thus α2,3-sialic acid is lactonized by intramolecular dehydration and at the same time α2,6-sialic acid is reacted with a nucleophilic agent such as alcohol or amine. Thus, molecules different in mass are produced depending on the linking mode of sialic acid, and therefore the linking mode of sialic acid can be distinctively examined by mass spectrometry. NPL 1 discloses a method for preparing a specimen to be used for mass spectrometry, in which a solution including isopropylamine and a dehydration-condensation agent is added to a released N-linked sugar chain, to lactonize α2,3-sialic acid and amidate α2,6-sialic acid.

### CITATION LIST

### NON PATENT LITERATURE

NPL 1: Nishikaze T., Tsumoto H., Sekiya S., Iwamoto S., Miura Y., Tanaka K., Analytical Chemistry, 2017, 89, 2353-2360.

### SUMMARY OF INVENTION

### TECHNICAL PROBLEM

The present inventors have found that, when the linking mode of sialic acid is examined, the accuracy of discrimination between α2,6-sialic acid and α2,3-sialic acid may be deteriorated. The reason for this is considered because, when an O-linked sugar chain released from glycoprotein is subjected to modification (amidation) that is linking mode-specific for sialic acid, α2,6-sialic acid linked to N-acetylgalactosamine (GalNAc) at a reducing end of the O-linked sugar chain is not correctly modified and a reaction that is partially the same as that of α2,3-sialic acid progresses. The present invention has been made in view of the above circumstances, and an object thereof is to enable α2,6-sialic acid and α2,3-sialic acid added to an O-linked sugar chain to be distinctly modified, and to release an O-linked sugar chain subjected to linking mode-specific modification, from glycoprotein, and analyze the O-linked sugar chain with good accuracy.

### SOLUTION TO PROBLEM

The present invention relates to
a method for analyzing an O-linked sugar chain subjected to linking mode-specific modification of sialic acid, the method including
a modification step of adding a modifier that subjects the O-linked sugar chain to linking mode-specific modification of sialic acid, to a sample including a glycoprotein to which the O-linked sugar chain is linked,
a releasing step of releasing the O-linked sugar chain subjected to linking mode-specific modification of sialic acid, from the glycoprotein, and
an analysis step of analyzing the released O-linked sugar chain subjected to linking mode-specific modification of sialic acid.

### ADVANTAGEOUS EFFECTS OF INVENTION

According to the present invention, it is possible to distinctly modify α2,6-sialic acid and α2,3-sialic acid added to an O-linked sugar chain, and to release an O-linked sugar chain subjected to linking mode-specific modification, from glycoprotein, and analyze the O-linked sugar chain with good accuracy.

### BRIEF DESCRIPTION OF DRAWINGS

Fig. 1 is a flowchart showing an analysis method according to one embodiment of the present invention.
Fig. 2 is each mass spectrum of sugar chains included in serum- and cerebrospinal fluid-derived glycoproteins in Experiment 1. I.S. represents an internal standard.
Fig. 3 shows an upper diagram that is a mass spectrum of a sugar chain included in a serum-derived glycoprotein, in Experiment 2, and a lower diagram that is a mass spectrum of a sugar chain included in a serum-derived glycoprotein, in Experiment 3.
Fig. 4 shows an upper diagram that is a mass spectrum of a sugar chain in sugar chain modification conducted by an MTT method with a glycoprotein having an O-linked sugar chain, in Experiment 4, and a lower diagram that is a mass spectrum of a sugar chain in linking mode-specific modification of sialic acid, conducted after sugar chain release from a glycoprotein having an O-linked sugar chain, in Experiment 4.
Fig. 5 is a mass spectrum of a sugar chain released by a β-elimination reaction after a glycoprotein having an O-linked sugar chain is subjected to linking mode-specific modification of sialic acid, in Experiment 5.
Fig. 6 is a graph showing the proportion of sialyl T not methylamidated during the change in concentration of methylamine, in Experiment 5.
Fig. 7 includes an upper diagram that is a graph showing the amount of recovery of an O-linked sugar chain (vertical axis: relative value to internal standard) during the change in pH in a β-elimination reaction, in Experiment 6, and a lower diagram that is a graph showing the type and the proportion of presence of a sugar chain recovered during the change in pH in a β-elimination reaction, in Experiment 6.
Fig. 8 includes each graph showing the proportion of a decomposed product of sialyl T methylamidated during the change in pH in a β-elimination reaction, in Experiment 6. A larger graph and a smaller graph are different only in scale of the vertical axis, and show the data of the same experiment. The smaller graph shows the upper limit values with respect to Entry 3 to Entry 5, in the vertical axis, but the larger graph shows the real proportions.
Fig. 9 includes each graph showing the proportion of a peeling reaction during the change in pH in a β-elimination reaction, in Experiment 6. A larger graph and a smaller graph are different only in scale of the vertical axis, and show the data of the same experiment. The smaller graph shows the upper limit values with respect to Entry 3 to Entry 5, in the vertical axis, but the larger graph shows the real proportions.

### DESCRIPTION OF EMBODIMENTS

A method for analyzing an O-linked sugar chain subjected to linking mode-specific modification of sialic acid, according to one embodiment of the present invention, includes
a modification step of adding a modifier that subjects the O-linked sugar chain to linking mode-specific modification of sialic acid, to a sample including a glycoprotein to which the O-linked sugar chain is linked,
a releasing step of releasing the O-linked sugar chain subjected to linking mode-specific modification of sialic acid, from the glycoprotein, and
an analysis step of analyzing the released O-linked sugar chain subjected to linking mode-specific modification of sialic acid.

When a dehydration-condensation agent is added to a sugar chain in order to conduct linking mode-specific modification of sialic acid, not only α2,3-sialic acid, but also α2,6-sialic acid to be amidated may be lactonized in a released O-linked sugar chain, thereby making distinction between α2,3-sialic acid and α2,6-sialic acid difficult. The present inventors have found that a sugar chain not released from protein can be subjected to a lactonization reaction, to suppress unexpected lactonization of α2,6-sialic acid and differently modify α2,6-sialic acid from α2,3-sialic acid. If modification of sialic acid, which is linking mode-specific, can be achieved in an O-linked sugar chain, the accuracy of discrimination of the linking mode of sialic acid can be enhanced. Even in a case where a sugar chain is an N-linked sugar chain, such a sugar chain not released from protein can be subjected to a lactonization reaction, to conduct different modification of sialic acid between α2,3-sialic acid and α2,6-sialic acid. According to the present invention, even if glycoprotein contains an O-linked sugar chain, sialic acid can be modified in a linking mode-specific manner, and the linking mode of sialic acid can be discriminated with good accuracy. Here, the "linking mode-specific modification of sialic acid" and "modification of sialic acid in a linking mode-specific manner" mean a modification reaction acting on sialic acid, in which a chemical structure produced by the modification reaction differs between a case where the sialic acid is α2,3-sialic acid and a case where the sialic acid is α2,6-sialic acid.

Sialic acid is positioned at a sugar chain end to be easily recognized from other molecule, and therefore the linking mode of sialic acid can be discriminated to clarify virus infection, or interaction between proteins on a cell surface. It is also known that the linking mode of sialic acid in glycoprotein is changed according to cancerization, and it is also expected that sialic acid is utilized as a biomarker for cancer. The effect of biopharmaceuticals differs depending on sugar chain modification, and therefore the linking mode of sialic acid can also be discriminated with good accuracy to assist quality control of biopharmaceuticals.

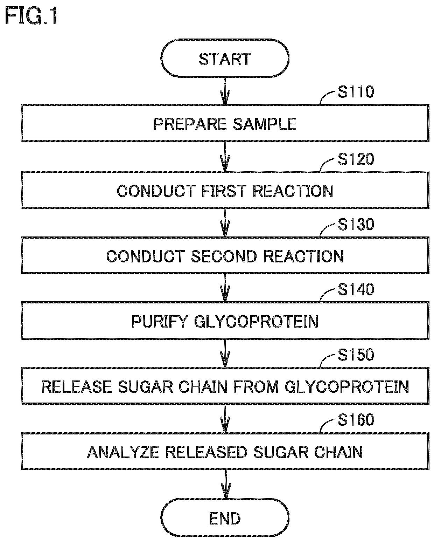

A flowchart showing a flow of an analysis method according to one embodiment of the present invention is shown in Fig. 1. In Step S110, a sample is prepared.

### (Sample)

A sample to be analyzed is not particularly limited, and may be organism- or cell-derived. The sample contains glycoprotein, and may further contain a released sugar chain or glycolipid. The analysis method according to the present invention is suitably used for examination of the linking mode of sialic acid in a sugar chain. Therefore, the sample preferably contains a sugar chain capable of having sialic acid at an end, more preferably contains a glycoprotein to which the sugar chain is linked. The analysis method according to the present invention can solve the problem of α2,6-sialic acid that may be lactonized in an O-linked sugar chain. Therefore, the sugar chain in the sample preferably contains an O-linked sugar chain, more preferably contains an O-linked sugar chain provided with α2,6-sialic acid. In other words, the sample preferably contains a glycoprotein to which such a sugar chain is linked. The analysis method according to the present invention is suitably used in a case where the sample contains a glycoprotein to which an O-linked sugar chain is linked, and the sample may also contain a glycoprotein to which an N-linked sugar chain is linked. The sample preferably contains no released O-linked sugar chain from the viewpoint of an enhancement in accuracy of linking mode-specific modification of sialic acid.

Herein, the "protein" refers to a collective term of a molecule in which 2 or more amino acids form a peptide linkage. Herein, the "protein" may encompass a peptide (including oligopeptide and polypeptide.) in which the number of amino acid residues is small, for example, the number of amino acid residues is less than 50, and a protein in which the number of amino acid residues is large, for example, the number of amino acid residues is 50 or more. Herein, the "glycoprotein" may include glycopeptide.

A glycoprotein in which the number of amino acid residues in a peptide chain is large may be cleaved at the peptide chain by an enzyme or the like. For example, in a case where a specimen for mass spectrometry is prepared, the number of amino acid residues in a peptide chain is preferably 30 or less, more preferably 20 or less, further preferably 15 or less. In a case where it is demanded that the origin of a peptide to which a sugar chain is linked is identified, the number of amino acid residues in a peptide chain is preferably 2 or more, more preferably 3 or more.

The digestive enzyme used in the case of cleavage of a peptide chain in glycoprotein is, for example, trypsin, Lys-C, arginine endopeptidase, chymotrypsin, pepsin, thermolysin, proteinase K, or pronase E. Two or more of these digestive enzymes may be used in combination. Conditions in cleavage of such a peptide chain are not particularly limited, and an appropriate protocol depending on the digestive enzyme to be used is adopted. Before cleavage of such a peptide chain, the protein in the sample may be subjected to denaturation treatment or alkylation treatment. Conditions of such denaturation treatment or alkylation treatment are not particularly limited. The cleavage treatment of such a peptide chain may be conducted before and after a first reaction or after a second reaction, described below. Such a peptide chain may be cleaved by not enzymatic cleavage, but chemical cleavage or the like.

Treatment for blocking an amino group in glycoprotein in the sample may be appropriately conducted. Examples of such treatment include dimethylamidation and guanidinylation of glycoprotein. Thus, a side reaction such as intramolecular dehydration condensation can be suppressed which can be caused with an amino group or a carboxy group located at an end of a main chain of protein during the first reaction or second reaction conducted.

### <Modification step>

In the modification step, a modifier that subjects an O-linked sugar chain to linking mode-specific modification of sialic acid is added. The linking mode-specific modification of sialic acid preferably includes a first reaction that lactonizes sialic acid. The linking mode-specific modification of sialic acid preferably further includes a second reaction that amidates a lactone structure obtained by the first reaction. In one mode of the present embodiment, the modifier preferably contains a lactonization reaction solution and an amidation reaction solution. The lactonization reaction solution and the amidation reaction solution are described below.

### (First reaction)

In Step S120, a glycoprotein to which a sugar chain is linked is subjected to a first reaction that lactonizes sialic acid. In the first reaction, in a case where sialic acid is linked to the glycoprotein, such sialic acid is selectively lactonized depending on the linking mode. In the first reaction, preferably not only α2,3-sialic acid, but also α2,8-sialic acid and α2,9-sialic acid, in the sugar chain, are lactonized.

The first reaction can be conducted with the sample being in contact with a solution for conducting linking mode-specific lactonization of sialic acid (hereinafter, also called lactonization reaction solution.). One example of the contacting method may include adding such a lactonization solution to the sample. The lactonization reaction solution preferably contains a dehydration-condensation agent. It is preferred to subject α2,6-sialic acid to modification different from lactonization, preferably amidation, at the same time as the first reaction. In this case, the lactonization reaction solution preferably further contains a nucleophilic agent containing at least one selected from the group consisting of alcohol, amine and any salt thereof, in addition to the dehydration-condensation agent.

The types and the concentrations of the dehydration-condensation agent and the nucleophilic agent may be adjusted so that a dehydration reaction or a nucleophilic reaction selectively occurs based on the linking mode of sialic acid. A lactone that is generated by intramolecular dehydration of a carboxy group of α2,3-sialic acid is a six-membered ring, and a lactone that can be generated by intramolecular dehydration of a carboxy group of α2,6-sialic acid is a seven-membered ring. α2,3-sialic acid that generates a six-membered ring more stable than a seven-membered ring is more easily lactonized than α2,6-sialic acid. The carboxy group of α2,3-sialic acid is located at a position where steric hindrance is relatively large as compared with the carboxy group of α2,6-sialic acid, and therefore a large molecule hardly reacts with α2,3-sialic acid as compared with α2,6-sialic acid. The types and the concentrations of the dehydration-condensation agent and the nucleophilic agent are adjusted so that different modification is made depending on the linking mode of sialic acid based on the difference in molecule structure due to the linking mode of sialic acid.

The dehydration-condensation agent preferably contains carbodiimide. The reason for this is because use of carbodiimide more hardly causes amidation of a carboxy group present at a site large in steric hindrance as compared with the case of use of a phosphonium-based dehydration-condensation agent (so-called BOP reagent) or a uronium-based dehydration-condensation agent as the dehydration-condensation agent. Examples of the carbodiimide include N,N'-dicyclohexylcarbodiimide (DCC), N-(3-dimethylaminopropyl)-N'-ethylcarbodiimide (EDC), N,N'-diisopropylcarbodiimide (DIC), 1-tert-butyl-3-ethylcarbodiimide (BEC), N,N'-di-tert-butylcarbodiimide, 1,3-di-p-toluylcarbodiimide, bis (2,6-diisopropylphenyl) carbodiimide, bis (trimethylsilyl) carbodiimide, 1,3-bis (2,2-dimethyl-1,3-dioxolan-4-ylmethyl) carbodiimide (BDDC) and salts thereof (hydrochloride and the like).

A highly nucleophilic additive is preferably used in addition to the carbodiimide in order to promote dehydration-condensation by the dehydration-condensation agent and suppress a side reaction. As the highly nucleophilic additive, 1-hydroxybenzotriazole (HOBt), 1-hydroxy-7-aza-benzotriazole (HOAt), 4-(dimethylamino) pyridine (DMAP), ethyl 2-cyano-2-(hydroxyimino) acetate (Oxyma), N-hydroxy-succinimide (HOSu), 6-chloro-1-hydroxy-benzotriazole (Cl-HoBt), N-hydroxy-3,4-dihydro-4-oxo-1,2,3-benzotriazine (HOOBt), or the like is preferably used.

The amine used as the nucleophilic agent preferably contains primary and/or secondary alkylamine(s) containing two or more carbon atoms. The primary alkylamine is preferably ethylamine, propylamine, isopropylamine, butylamine, sec-butylamine, tert-butylamine, or the like. The secondary alkylamine is preferably dimethylamine, ethylmethylamine, diethylamine, propylmethylamine, isopropylmethylamine, or the like. An amine having a branched alkyl group, such as isopropylamine, is preferably used from the viewpoint of hardly causing amidation of a carboxy group present at a site large in steric hindrance, such as a carboxy group of α2,3-sialic acid. In a case where the amine is used in the nucleophilic agent in the lactonization reaction solution, a carboxy group of some sialic acid such as α2,6-sialic acid is amidated based on the linking mode of sialic acid.

The alcohol used as the nucleophilic agent is not particularly limited, and, for example, methanol and ethanol can be used. In a case where the alcohol is used as the nucleophilic agent of the lactonization reaction solution, a carboxy group of some sialic acid such as α2,6-sialic acid is esterified based on the linking mode of sialic acid. The nucleophilic agent may contain a salt of the nucleophilic agent.

The concentration of the dehydration-condensation agent in the lactonization reaction solution is, for example, 1 mM or more and 5 M or less, preferably 10 mM or more and 3 M or less. In a case where the carbodiimide and the highly nucleophilic additive such as Oxyma, HOAt or HOBt are used in combination, each concentration thereof is preferably within the above range. The concentration of the nucleophilic agent in the lactonization reaction solution is, for example, 0.01 M or more and 20 M or less, preferably 0.1 M or more and 10 M or less. The reaction temperature of the first reaction may be around -20°C or more and 100°C or less, preferably -10°C or more and 50°C or less.

The first reaction can be carried out even in a liquid phase or in a solid phase. In a case where the reaction is conducted in a liquid phase, the reaction is preferably conducted in a non-aqueous solvent such as dimethyl sulfoxide (DMSO) or dimethylformamide (DMF). When the reaction is conducted in the non-aqueous solvent, a side reaction tends to be suppressed. The concentration of each component in the liquid phase reaction is not particularly limited, and can be appropriately determined depending on the types and the like of the dehydration-condensation agent and the amine.

In a case where the first reaction is conducted in a solid phase, a solid-phase carrier is not particularly limited as long as the glycoprotein can be fixed. In order to fix the glycoprotein, for example, a solid-phase carrier having an epoxy group, a tosyl group, a carboxy group, or an amino group as a ligand can be used. A solid-phase carrier having a hydrazide group, an aminooxy group or the like as a ligand may be used to fix a sugar chain-containing glycoprotein. The reaction is conducted in the state of the sample being fixed to the solid-phase carrier, and thus removal of the reaction solution after the reaction is facilitated and modification of sialic acid can be efficiently conducted. In a case where magnetic beads are used in the solid-phase carrier, magnetic beads to which the glycoprotein is linked can be collected by a magnet to remove an excess reagent, or the beads can be washed with a solvent. In a case where a resin is used in the solid-phase carrier, the resin may be recovered after filtration for removal of an excess reagent, or the resin may be precipitated by a centrifugal operation to remove an excess reagent in the supernatant. In a case where the first reaction is conducted in a liquid phase, an excess reagent may be removed by ultrafiltration.

The sample after the first reaction may be, if necessary, subjected to treatment such as purification, desalting, solubilization, concentration, and drying by a known method, to result in removal of the lactonization reaction solution or a reduction in concentration of the lactonization reaction solution.

### (Second reaction)

In Step S130, a second reaction that amidates a lactone structure generated by the first reaction is preferably conducted. The second reaction may be conducted by contacting the sample after the first reaction, with a solution for amidation (hereinafter, also called amidation reaction solution). Although the first reaction can distinctly modify an α2,3-linkage and an α2,6-linkage of sialic acid, a lactone structure generated from α2,3-sialic acid may be unstable and may be returned to an original carboxylic acid structure by hydrolysis. The second reaction can specifically amidate the lactone structure to stabilize the lactone structure. The second reaction can simultaneously allow sialic acid to be modified differently in terms of mass, in a linking mode-specific manner, thereby distinguishing α2,3-sialic acid and α2,6-sialic acid. The second reaction can be conducted to more specifically and rapidly modify sialic acid in a linking mode-specific manner. One example of the second reaction may be aminolysis. Aminolysis is a reaction different from hydrolysis because of being a reaction based on interaction between an amino group and lactone, and being suitably conducted even under an anhydrous condition. Herein, ring-opening and amidation of lactone with ammonia, amine or any salt thereof, which can be made even under an anhydrous condition, are called aminolysis. Another example of the second reaction may be amidation of the lactone structure with a strong dehydration-condensation agent. Examples of the dehydration-condensation agent used in the second reaction include a phosphonium-based condensation agent and a uronium-based condensation agent, and specific examples thereof include benzotriazole-1-yloxytris (dimethylamino) phosphonium hexafluorophosphate (BOP), benzotriazole-1-yloxytris (pyrrolidino) phosphonium hexafluorophosphate (PyBOP), 2-(1H-benzotriazole-1-yl)-1,1,3,3-tetramethyluronium hexafluorophosphate (HBTU), 2-(7-aza-1H-benzotriazole-1-yl)-1,1,3,3-tetramethyluronium hexafluorophosphate (HATU), (1-[(1-(cyano-2-ethoxy-2-oxoethylideneaminooxy)dimethylaminomorpholino)] uronium hexafluorophosphate (COMU), 2-(1H-benzotriazole-1-yl)-1,1,3,3-tetramethylaminium tetrafluoroborate (TBTU), tetramethylfluoroformamidinium hexafluorophosphate (TFFH), and (7-azabenzotriazole-1-yloxy) tripyrrolidinophosphonium hexafluorophosphate (PyAOP). A ring-opening operation of lactone may be inserted before amidation with such a strong dehydration-condensation agent.

The amidation reaction solution preferably contains at least one selected from the group consisting of ammonia, amine and a salt thereof. In a case where the amine is used, it is preferable to use a different amine from the amine used in the lactonization reaction solution, or change the mass by, for example, modification with a stable isotope. Different amines in mass can be used respectively in the first reaction and the second reaction, to amidate sialic acid so that the mass differs depending on the linking mode. In one mode of the present embodiment, the second reaction is preferably an aminolysis reaction. No dehydration-condensation agent is necessary for aminolysis, and the amidation reaction solution may contain no dehydration-condensation agent. The amidation reaction solution may contain a dehydration-condensation agent, and the amidation reaction solution can be prepared by, for example, adding ammonia, amine or a salt thereof without removal of the lactonization reaction solution added to the sample in the first reaction. The second reaction can stabilize the lactone structure by such a simple operation.

The amine contained in the amidation reaction solution is preferably a primary amine, more preferably a primary amine having a straight hydrocarbon group, further preferably a primary amine having a straight alkyl group. The amine contained in the amidation reaction solution is, as the primary amine having a straight alkyl group, preferably a primary amine having 10 or less carbon atoms, more preferably a primary amine having 7 or less carbon atoms, further preferably methylamine, ethylamine, propylamine, butylamine or pentylamine, most preferably methylamine. The amine contained in the amidation reaction solution preferably has a straight structure not having any branch (hereinafter, "branch" indicates a branch of a hydrocarbon chain), or has a small number of carbon atoms, from the viewpoint of more efficient amidation of the lactone structure.

In a case where the amine contained in the amidation reaction solution is a primary amine having an unsaturated linear hydrocarbon group, the unsaturated linear hydrocarbon group preferably contains a double bond, the unsaturated linear hydrocarbon group more preferably contains an allyl group, and the amine is preferably allylamine. The amine contained in the amidation reaction solution may be a primary amine containing a hydroxy group, or may be ethanolamine. The amine contained in the amidation reaction solution may contain various functional groups other than an alkyl group. The amidation reaction solution may contain a salt of the above amine. As a result of the amidation reaction, a sugar chain is modified so as to contain such a functional group, and thus the modified sugar chain is more easily separated by not only mass spectrometry, but also chromatography or the like.

The concentration of the ammonia, amine and salt thereof in the amidation reaction solution is preferably 0.1 M or more, more preferably 0.3 M or more, further preferably 0.5 M or more, further preferably 1.0 M or more, most preferably 3.0 M or more. As a suitable example, the amidation reaction solution contains ammonia or a primary amine, particularly methylamine, the concentration of the ammonia or primary amine such as methylamine is preferably 0.1 M or more, more preferably 0.3 M or more, further preferably 0.5 M or more, further preferably 1.0 M or more, most preferably 3.0 M or more. As the concentration of the ammonia, amine and salt thereof in the amidation reaction solution is higher, the lactone structure can be more efficiently amidated. The upper limit value of the concentration of the ammonia, amine and salt thereof in the amidation reaction solution is not particularly restricted, and may be, for example, 16 M or less.

The solvent of the amidation reaction solution may be an aqueous solvent or an organic solvent, and the water content is preferably lower from the viewpoint that hydrolysis of lactone is prevented to allow rapid amidation to certainly occur. The solvent of the amidation reaction solution is preferably a dehydration solvent to which a dehydration operation for decreasing the water content is applied, further preferably an anhydrous solvent. The solvent of the amidation reaction solution preferably contains at least one of methanol and acetonitrile. The amidation reaction solution may contain water, or the solvent of the amidation reaction solution may be water.

In a case where the amidation reaction is aminolysis, the solution thereof preferably has a pH of 7.7 or more, more preferably a pH of 8.0 or more, further preferably a pH of 8.8 or more, most preferably a pH of 10.3 or more. If the pH of the amidation reaction solution is higher, the lactone structure can be more efficiently amidated. The upper limit value of the pH of the amidation reaction solution is not particularly restricted, and may be, for example, 14 or less. In a case where a strong dehydration-condensation agent is used to amidate the lactone structure, the pH thereof is not particularly limited and the pH may be adjusted depending on a dehydration-condensation agent used.

In a case where the second reaction is aminolysis, the reaction can be completed within several seconds to several minutes. The time for contacting the sample with the amidation reaction solution for amidation of the lactone structure is preferably less than 1 hour, more preferably less than 30 minutes, further preferably less than 15 minutes, further preferably less than 5 minutes, most preferably less than 1 minute. Suitably, the sample may be washed with the amidation reaction solution, or the amidation reaction solution may only temporarily flow to the sample that is supported on a carrier or the like. The lower limit value of the time for contacting the sample with the amidation reaction solution for amidation of the lactone structure is not particularly restricted, and may be, for example, 1 second or more. In a case where a strong dehydration-condensation agent is used to amidate the lactone structure, the reaction time is not particularly limited, and the reaction time may be adjusted depending on a dehydration-condensation agent used. For example, the reaction time is preferably 30 minutes or more, further preferably 1 hour or more, most preferably 2 hours or more. The time until the termination of contact between the sample and the amidation reaction solution after the termination of contact between the sample and the lactonization reaction solution is preferably less than an hour and a half, more preferably less than 1 hour, further preferably less than 30 minutes. The second reaction is completed in a short time, and therefore it is possible to prevent decomposition of an unstable lactone structure and thus loss of quantitativeness in examination of a sugar chain. The reaction time of the second reaction can be set to a short time, thereby more efficiently conducting examination of the sample. The lower limit value of the time until the termination of contact between the sample and the amidation reaction solution after the termination of contact between the sample and the lactonization reaction solution is not particularly restricted, and may be, for example, 1 second or more.

The second reaction can be conducted even in a liquid phase or in a solid phase. The state of the sample in the occurrence of the amidation reaction is not particularly limited as long as the sample and the amidation reaction solution can be contacted, and the amidation reaction solution is preferably contacted in the state where the glycoprotein is linked to or adsorbs to a solid-phase carrier. The solid-phase carrier used in the second reaction is not particularly limited as long as the glycoprotein can be fixed, and examples include a solid-phase carrier usable in the first reaction.

The sample after the second reaction may be, if necessary, subjected to treatment such as purification, desalting, solubilization, concentration, and drying by a known method, to result in removal of the amidation reaction solution or a reduction in concentration of the amidation reaction solution. Removal of an excess reagent after the second reaction can be conducted depending on the reaction method in the solid phase or liquid phase, by the method described in the section of the first reaction.

### (Purification of glycoprotein)

In Step S140, the glycoprotein is preferably purified. This step can remove the solution(s) used in the first reaction and/or the second reaction and enhance the efficiency of the downstream step. The purification method and conditions of the glycoprotein can be appropriately selected depending on the type, properties, molecular weight, and the like of the glycoprotein. The purification of the glycoprotein may be conducted by a protein precipitation method. As a precipitant, a salt such as ammonium sulfate; an organic solvent such as acetone, acetonitrile, and chloroform; alcohol such as methanol, propanol, and ethanol; acid such as trichloroacetic acid (TCA), hydrochloric acid, and metaphosphoric acid; a water-soluble polymer such as polyethylene glycol and dextran, and any combination thereof can be used. In the protein precipitation method, for example, the precipitant is added to a sample containing the glycoprotein, and left to still stand at -20°C or more and 30°C or less for a proper time. Thereafter, a precipitate generated by centrifugation of the sample left to still stand may be recovered. The purification of the glycoprotein may be conducted with the glycoprotein being immobilized on the solid-phase carrier, and gel filtration chromatography, ion-exchange chromatography, hydrophobic chromatography, affinity chromatography, and/or the like may be utilized.

### <Releasing step>

In Step S150, a sugar chain is released from the glycoprotein. As the method for releasing a sugar chain from the glycoprotein, enzyme treatment with O-glycosidase, N-glycosidase, endoglycoceramidase, or the like, and a chemical releasing method such as hydrazine decomposition or β-elimination can be used. A peptide chain of the glycoprotein may be cleaved before treatment for releasing a sugar chain. Modification such as labelling of a reducing end of a sugar chain with 3-aminoquinoline (3AQ), anthranilic acid (2AA), 1-phenyl-3-methyl-5-pyrazolone (PMP), or the like may be conducted together with release of a sugar chain.

In a case where an N-linked sugar chain is released from a peptide chain of the glycoprotein, enzyme treatment with peptide-N-glycosidase F (PNGase F), peptide-N-glycosidase A (PNGase A), endo-β-N-acetylglucosaminidase (Endo M), or the like is suitably used. In a case where an O-linked sugar chain is released from a peptide chain of the glycoprotein, a chemical releasing method is suitably used. A hydrazine decomposition method may be conducted according to a known method, and, for example, anhydrous hydrazine or hydrous hydrazine is added to a sample containing the glycoprotein, and heated. A β-elimination method may be conducted according to a known method, and, for example, ammonium carbamate, saturated ammonia, ammonium carbonate, anhydrous trifluoromethanesulfonic acid (anhydrous TFMS), sodium hydroxide, dimethylamine, or the like is added to a sample containing the glycoprotein, and then heated under an alkaline condition. It is preferable from the viewpoint of suppression of a side reaction by peeling to release an O-linked sugar chain with a powder of an ammonium salt such as ammonium carbamate. Such a side reaction by peeling may also be inhibited by allowing a pyrazolone reagent such as PMP to coexist during the reaction under an alkaline condition, and releasing and at the same time labelling an O-linked sugar chain. The pH in the β-elimination reaction is preferably a pH of 11.0 or less, more preferably a pH of 10.0 or less from the viewpoint of prevention of conversion of sialic acid methylated in the second reaction, into carboxylic acid, and from the viewpoint of the release efficiency of an O-linked sugar chain. The pH in the β-elimination reaction is preferably a pH of 9.5 or less from the viewpoint of decomposition of a sugar chain sialylated and suppression of the peeling reaction. The pH in the β-elimination reaction conducted is usually a pH of 7.5 or more.

Release of a sugar chain may be conducted in a liquid phase or may be conducted in a solid phase. For example, a sugar chain may be recovered by subjecting a glycoprotein immobilized to the solid-phase carrier, to the above cleavage treatment of a sugar chain. A sugar chain may also be recovered by subjecting sugar chain protein linked to a solid-phase carrier having a hydrazide group, to cleavage treatment of a sugar chain, and then releasing a sugar chain with a weakly acidic solution. The reaction is conducted in the state of the sample being immobilized to the solid-phase carrier, and thus removal of the reaction solution or desalting purification is more facilitated and preparation of the sample can be simplified.

A released sugar chain may be purified according to a known method, and protein and such a sugar chain may be separated by, for example, liquid-liquid extraction with an organic solvent such as chloroform. The released sugar chain can also be simply purified by a solid-phase carrier (column). The released sugar chain can also be recovered by linkage to a solid-phase carrier having a hydrazide group or an aminooxy group. Examples of the solid-phase carrier having a hydrazide group include "BlotGlyco" manufactured by Sumitomo Bakelite Co., Ltd. The sugar chain may also be purified by adsorption thereof to a carrier for hydrophilic interaction chromatography (hereinafter, also called HILIC). The carrier for HILIC preferably includes an amide group. The released sugar chain may also be purified with not only a carbon column, but also a carrier of a reversed-phase system, such as C18. The sugar chain, which is labelled, may also be purified by adsorption thereof to carbon or C18.

### <Analysis step>

In Step S160, the released sugar chain is analyzed. The sugar chain to be analyzed preferably contains a released O-linked sugar chain subjected to linking mode-specific modification of sialic acid. The analysis method can be mass spectrometry, chromatography, electrophoresis or any combination thereof. The above lactonization reaction allows sialic acid to be modified in a linking mode-specific manner, and the mass differs between a sugar chain to be hardly lactonized, such as α2,6-sialic acid, and a sugar chain to be easily lactonized, such as α2,3-sialic acid, α2,8-sialic acid, and α2,9-sialic acid. Accordingly, these sugar chains can be separated depending on the linking mode of sialic acid by mass spectrometry, chromatography, electrophoresis, or the like.

The ionization method in mass spectrometry is not particularly limited, a matrix-assisted laser desorption/ionization (MALDI) method, an electrospray (ESI) method, a nano-electrospray ionization (nano-LSI) method, or the like can be used, and a MALDI method is preferred. Any of a positive ion mode and a negative ion mode may be used for ionization in mass spectrometry. Such mass spectrometry may be conducted in a multistage manner. Thus, the structure of a sugar chain or the structure of a peptide chain, other than the linking mode of sialic acid, can be suitably examined.

The linking mode of sialic acid may be analyzed based on properties of a modified body resulting from the first reaction and the second reaction by an analysis method other than mass spectrometry, such as chromatography or electrophoresis. The column used in liquid chromatography is not particularly limited, and a hydrophobic reversed-phase column such as C30, C18, C8, and C4, a carbon column, a normal-phase column for HILIC, and the like can be appropriately used. It is preferable for more precise analysis conducted of each component in the sample to conduct liquid chromatography and then conduct measurement by mass spectrometry. In this case, an eluted liquid from liquid chromatograph is more preferably ionized directly by ESI or the like in a mass spectrometer by online control. Such electrophoresis may be an SDS-PAGE method, a capillary electrophoresis method, microchip electrophoresis, a two-dimensional electrophoresis method, or the like, and capillary electrophoresis is preferred.

### EXAMPLES

Hereinafter, the present invention is described in more detail with reference to Examples, but the present invention is not limited to these Examples. The designation "%" indicates "% by mass" unless particularly noted.

### <Experiment 1>

First, the first reaction and the second reaction were conducted without release of any sugar chain from the glycoprotein. Next, protein precipitation was conducted to remove an excess reagent. Thereafter, an O-linked sugar chain was released from the glycoprotein, and the sugar chain was analyzed by mass spectrometry. A specific procedure is shown below.
1) Each of freeze-dried products of a serum and a cerebrospinal fluid was prepared as a sample containing the glycoprotein.
2) One hundred µL of a lactonization reaction solution containing 2 M isopropylamine hydrochloride (nucleophilic agent), 500 mM N-(3-dimethylaminopropyl)-N'-ethylcarbodiimide hydrochloride (dehydration-condensation agent), 500 mM 1-hydroxybenzotriazole monohydrate (highly nucleophilic additive) and dimethyl sulfoxide as a solvent was added to the sample, and reacted at room temperature for 1 hour (first reaction).
3) One hundred µL of 10% methylamine was added and stirred (second reaction).
4) Eight hundred µL of a solution containing 2.5% trifluoroacetic acid (TFA) and acetonitrile (ACN) as a solvent was added to precipitate protein.
5) The supernatant was removed and the protein precipitated was recovered.
6) Dithiothreitol (DTT) was added for reduction alkylation of the glycoprotein.
7) Precipitation by ethanol was performed to purify protein.
8) A sugar chain was released from the glycoprotein, and labelled. Fifty µL of a solution containing a final concentration 0.14 M of sodium hydroxide, a final concentration 0.2 M of 1-phenyl-3-methyl-5-pyrazolone (PMP) and 40% methanol was added, and reacted at 85°C for 16 hours.
9) Chloroform was added and vigorously stirred and liquid-liquid extraction was conducted to dispose a chloroform layer. This operation was repeated three times to remove PMP from the sample.
10) An aqueous layer including the sugar chain was recovered, and the sugar chain was purified with a graphite carbon column ("Supelclean^{™} ENVI-Carb^{™} Bulk Packing" manufactured by SPELCO). After the column was washed with water, 10% ACN, 0.05% TFA, and 20% ACN, 0.05% TFA, elution was made with 50% ACN, 0.05% TFA.
11) An eluted product was concentrated under reduced pressure, and purified with iatro beads (normal-phase carrier, based on silica). Specifically, a sample prepared so that 95% ACN was achieved was adsorbed to the carrier, washed with 95% ACN, 1% acetic acid, and 98% ACN, 2% acetic acid, and then eluted with 50% ACN.
12) The eluted product was dried, and then re-dissolved in water, to prepare a specimen for analysis. A 2,5-dihydroxybenzoic acid (DHB) matrix was used to conduct mass spectrometry in a positive ion mode by time-of-flight mass spectrometry (MALDI-TOF-MS) (product name "Ultraflex II" manufactured by Bruker).

Herein, 2) and 3) described above correspond to the modification step and 8) to 11) described above correspond to the releasing step. In addition, 12) described above corresponds to the analysis step.

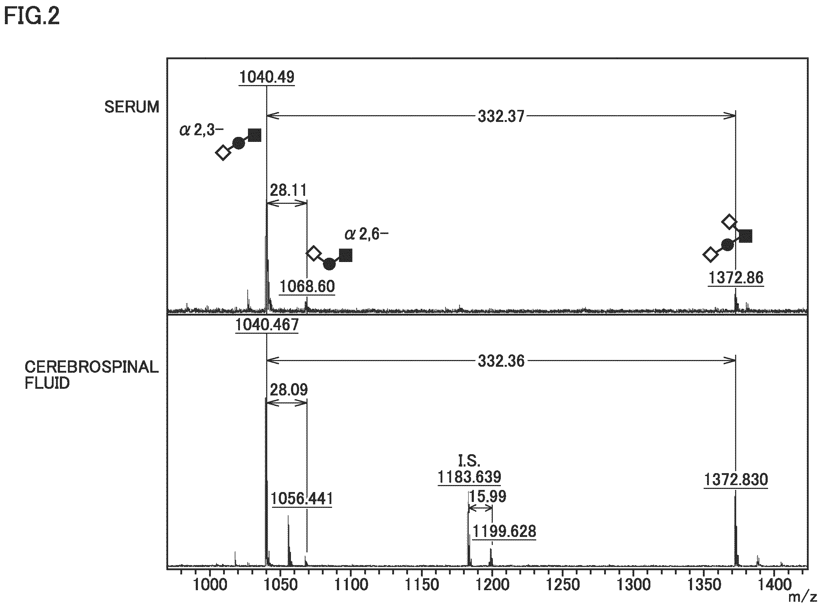

Each mass spectrum of sugar chains included in serum- and cerebrospinal fluid-derived glycoproteins is shown in Fig. 2. A difference of about 28 Da was observed between a sugar chain having α2,3-sialic acid (m/z 1040.49) and a sugar chain having α2,6-sialic acid (m/z 1068.60), and it was indicated that sialic acid was modified by linking mode-specific modification and could be distinguished by mass spectrometry. Although a peak at m/z 1372.86, assigned to α2,3-sialic acid methylamidated and α2,6-sialic acid isopropylamidated, was confirmed with respect to a diallyl sugar chain having both α2,3-sialic acid and α2,6-sialic acid, no peak was observed between this peak and a position smaller than this peak by 28 Da. Accordingly, it was confirmed that α2,6-sialic acid was not lactonized and α2,3-sialic acid and α2,6-sialic acid were modified differently.

### <Experiment 2>

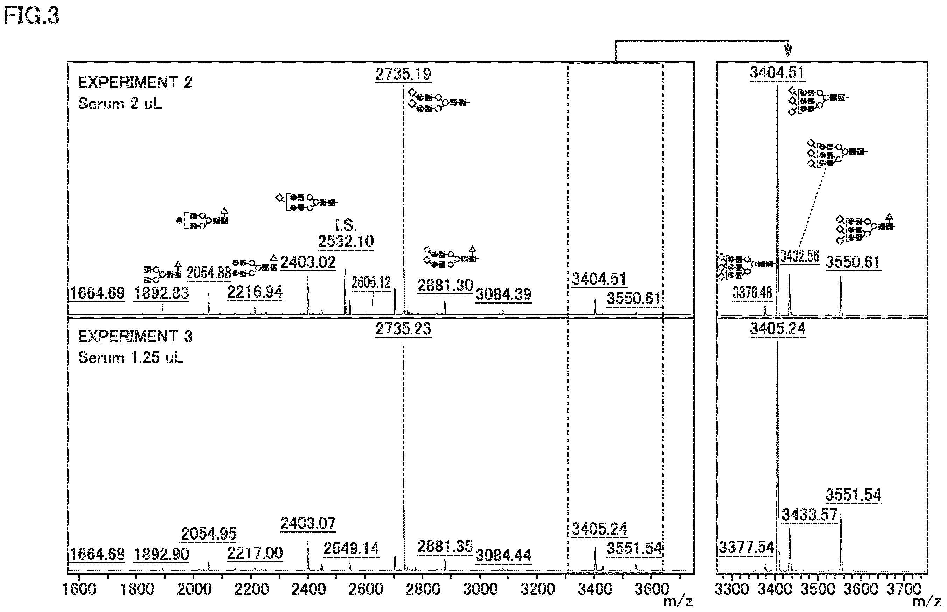

As a comparative experiment, an N-linked sugar chain was released from the glycoprotein, the released sugar chain was recovered with BlotGlyco beads, the first reaction and the second reaction were conducted on a sugar chain linked to the beads to modify sialic acid, and thereafter mass spectrometry of the released sugar chain recovered from the beads was conducted. The resulting mass spectrum is shown in the upper diagram of Fig. 3.

### <Experiment 3>

First, the first reaction and the second reaction were conducted in a liquid phase without release of any sugar chain from the glycoprotein. Next, protein precipitation was conducted to remove an excess reagent. Thereafter, an N-linked sugar chain was released from the glycoprotein, and the sugar chain was analyzed by mass spectrometry. The resulting mass spectrum is shown in the lower diagram of Fig. 3. A specific procedure is shown below.
1) A freeze-dried product of a serum was prepared as a sample.
2) One hundred µL of a lactonization reaction solution containing 2 M isopropylamine hydrochloride (nucleophilic agent), 500 mM N-(3-dimethylaminopropyl)-N'-ethylcarbodiimide hydrochloride (dehydration-condensation agent), 500 mM 1-hydroxybenzotriazole monohydrate (highly nucleophilic additive) and dimethyl sulfoxide as a solvent was added to the sample, and stirred for 1 hour (first reaction).
3) One hundred µL of 10% methylamine was added and stirred (second reaction).
4) Eight hundred µL of a solution containing 2.5% trifluoroacetic acid (TFA) and acetonitrile (ACN) as a solvent was added to precipitate protein.
5) The supernatant was removed and the protein precipitated was recovered.
6) Dithiothreitol (DTT) was added for reduction alkylation of the glycoprotein.
7) Precipitation by ethanol was performed to purify protein.
8) The protein was digested by trypsin (10 mg/mL).
9) An N-linked sugar chain was released by PNGaseF (2U manufactured by Roche Diagnostics K.K.).
10) A solid-phase carrier ("BlotGlyco" manufactured by Sumitomo Bakelite Co., Ltd.) made of beads having a hydrazide group as a ligand was used to purify the N-linked sugar chain according to the protocol.
11) A reducing end of the sugar chain was labelled with a sensitizing reagent aoWR at the same time of release from the carrier, to prepare a specimen for analysis.
12) A DHB matrix was used to conduct mass spectrometry in a positive ion mode with MALDI-TOF-MS ("product name Ultraflex II" manufactured by Bruker).

As shown in Fig. 3, almost the same mass spectrum was obtained in Experiment 2 and Experiment 3. It was found for an N-linked sugar chain that sialic acid was modified by linking mode-specific modification in the same manner in a case where the first reaction and the second reaction were conducted on the released sugar chain and a case where the first reaction and the second reaction were conducted on the glycoprotein and thereafter the sugar chain was released, and these cases were distinguishable by mass spectrometry. It has been indicated by the present invention that linking specificity of sialic acid can be rapidly discriminated with good accuracy even in any case where a sugar chain of glycoprotein contains N-linked sialic acid or O-linked sialic acid.

### <Experiment 4>

There are shown as comparative experiments, an experiment in which a glycoprotein having an O-linked sugar chain was used and no linking mode-specific modification of sialic acid was conducted and an experiment in which linking mode-specific modification of sialic acid was conducted after release of a sugar chain. To Fetuin (100 µg) as the glycoprotein were added 20 µL of 50% hydroxylamine and 20 µL of 0.5 M sodium hydroxide, and reacted at 50°C for 1 hour to release a sugar chain. After completion of the reaction, 10 µL of 1 M hydrochloric acid was added for quenching, 200 µL of acetone was added, and the resultant was concentrated by a centrifugal evaporator. After the concentration, a concentrated product was re-dissolved in 50 µL of water, and a released sugar chain was captured with BlotGlyco beads. A methylesterification method (MTT method) or the first reaction and the second reaction (sialic acid-linking mode-specific alkylamidation method; herein also referred to as "SALSA method".) were conducted on a solid phase, to modify sialic acid. According to the MTT method, modification not specific for any linking mode was conducted, thereby esterifying all sialic acid. The first reaction and the second reaction were conducted by the same method as in Experiment 1. A reducing end of the sugar chain was labelled with a sensitizing reagent aoWR, and then purified with a HILIC column. The aoWR-labelled sugar chain purified was mixed with 2,5-dihydrobenzoic acid (10 mg/mL), and examination with MALDI-TOF-MS (product name "Ultraflex II" manufactured by Bruker) was conducted.

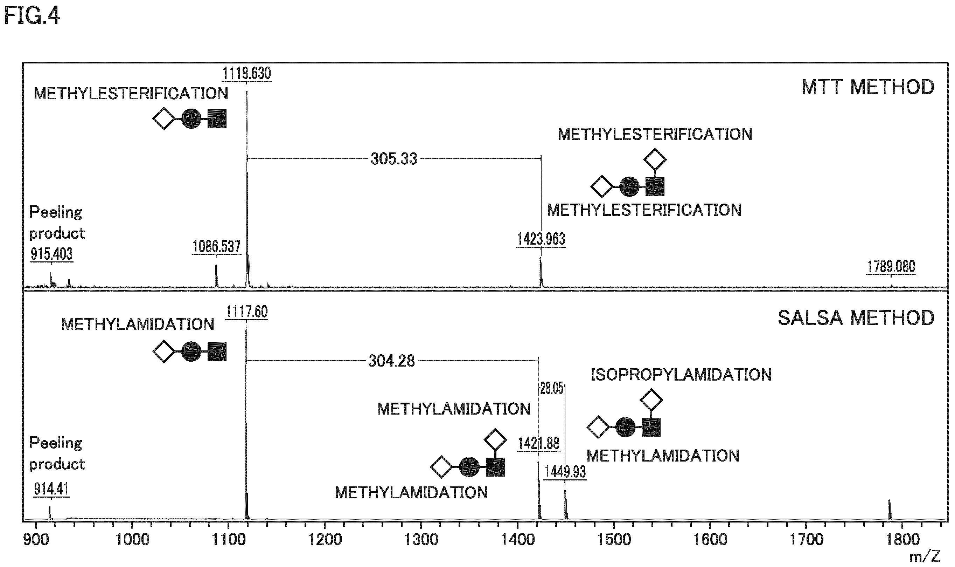

The resulting mass spectrum is shown in Fig. 4. The MTT method resulted in methylesterification of sialic acid, sialyl T was detected at m/z 1118.6 [M + H]⁺ and disialyl T was detected at m/z 1424.0 [M + H]⁺. In the SALSA method, sialyl T having α2,3-linked sialic acid was detected as a methylamide body at m/z 1117.6 [M + H]⁺. In this regard, a signal assigned to disialyl T having α2,3-linked sialic acid and α2,6-linked sialic acid, which modified with methylamide and isopropylamide, was observed at m/z 1449.8 [M + H]⁺, and α2,6-linked sialic acid modified with methylamide was also detected at m/z 1421.9 [M + H]⁺. Even in both the methods, no linking mode-specific modification of sialic acid, of an O-linked sugar chain, was achieved.

### <Experiment 5>

The concentration of methylamine was changed to research the efficiency of linking mode-specific modification of sialic acid, of an O-linked sugar chain. First, the first reaction and the second reaction were conducted on Fetuin (100 µg). Specifically, 100 µL of a lactonization reaction solution (2 M iPA-HCl, 500 mM EDC-HCl, 500 mM HOBt) was added to Fetuin, and stirred at room temperature for 1 hour (first reaction). Subsequently, 100 µL of an aqueous 10%, 16.6% or 40% methylamine solution was added to the reaction solution, and these were stirred (second reaction). Thereafter, 1 mL of acetonitrile containing 2.5% trifluoroacetic acid was added, and left to still stand at a temperature of -30°C for 1 hour. Centrifugation was made at 14000 g for 20 minutes, and the supernatant was removed to remove a SALSA reaction solution. Furthermore, 1 mL of acetonitrile was added to the residue and centrifuged at 14000 g for 20 minutes, to remove the supernatant. The resulting protein-containing residue was re-dissolved in 10 mL of water. A β-elimination reaction solution for release of a sugar chain from glycoprotein was prepared. The β-elimination reaction solution was a solution in which sodium hydroxide (NaOH) (final concentration 0.7 M) and 3-methyl-1-phenyl-5-pyrazolone (PMP) (final concentration 1 M) were dissolved in an aqueous 60% methanol solution. Forty µL of the β-elimination reaction solution was added to Fetuin in which sialic acid was modified in a linking mode-specific manner, and these were kept at a temperature of 85°C by a thermal cycler and reacted for 16 hours. After completion of the reaction, 50 pmol of bisPMP-labelled chitotetraose was added, and then neutralized with 10 µL of 4 M hydrochloric acid. Chloroform was added and vigorously stirred and liquid-liquid extraction was made. A chloroform layer was removed, and this was repeated three times. An aqueous layer including a sugar chain was recovered and purified with a graphite carbon column and iatro beads. The purification method was the same as in Experiment 1. A bisPMP-labelled sugar chain purified was mixed with 2,5-dihydrobenzoic acid (10 mg/mL) including 10% sodium 2,5-dihydrobenzate, and examined with MALDI-TOF.

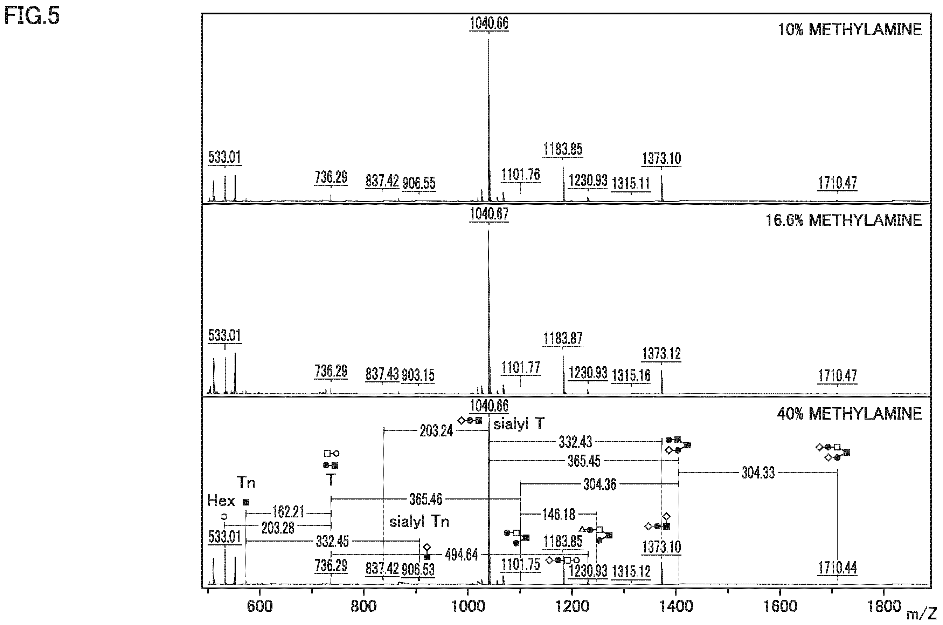

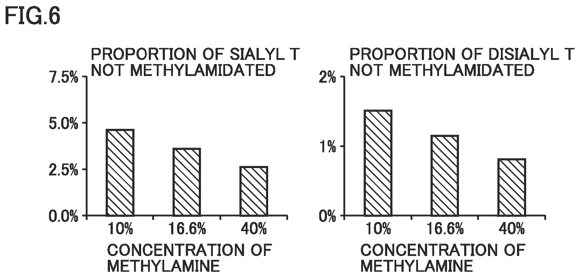

The result of a mass spectrum is shown in Fig. 5. In all the reactions, most of sialyl T having α2,3-linked sialic acid was observed at m/z 1040.7 [M + Na]⁺, and disialyl T having two, α2,3-linked sialic acid and α2,6-linked sialic acid, was observed at m/z 1373.1 [M + Na]⁺. It was confirmed that the sugar chain on the glycoprotein was directly subjected to the SALSA method and thus subjected to modification of sialic acid, and then released by the β-elimination reaction, thereby resulting in progression of linking mode-specific derivatization, and cleavage of the sugar chain itself derivatized by the β-elimination reaction, from the glycoprotein. Furthermore, the influence of the concentration of methylamine was studied. α2,3-linked sialyl T detected at m/z 1040.7 [M + Na]⁺, with modification by methylamidation, is detected at m/z 1027.6 [M + Na]⁺ in the case of unmodified carboxylic acid. The proportion of unmodified sialyl T relative to sialyl T modified by methylamidation was 4.6% in the case of 10% of methylamine, and 2.6% in the case of 40% of methylamine. Similarly, the proportion of unmodified disialyl T relative to disialyl T modified by methylamidation was 1.5% in the case of 10% of methylamine, and 0.8% in the case of 40% of methylamine (Fig. 6). As the concentration of amine was higher in the aminolysis reaction, the efficiency of methylamidation of sialic acid was higher.

### <Experiment 6>

The pH in the β-elimination reaction was changed to examine an O-linked sugar chain to be released. Human plasma (10 µL) was subjected to linking mode-specific modification of sialic acid, by the same method as in Experiment 5. An aqueous 40% methylamine solution was used in the second reaction. After glycoprotein was washed by the same method as in Experiment 5, 0.1 mL of water was added to a precipitate, and suspended with a water-bath-type ultrasonic treatment vessel. To the suspension was added 0.4 mL of a β-elimination reaction solution prepared in advance, and these were admixed. The β-elimination reaction solution was prepared by dissolving PMP (final concentration 1 M) and NaOH (final concentration 0.7 to 0.975 M) in a 60% acetonitrile solution. The concentration of NaOH was changed, and the pH was set to five types. The composition and the pH are shown in Table 1. A reaction was conducted at a temperature of 85°C for 16 hours, to subject an O-linked sugar chain to β-elimination and at the same time PMP labelling. After completion of the reaction, 0.4 mL of 4 M HCl was added for neutralization. An operation was repeated four times, in which 0.4 mL of chloroform was added and vigorously stirred and a chloroform layer was disposed by liquid-liquid extraction. An aqueous layer including a labelled sugar chain was recovered, and purified with a graphite carbon column and iatro beads. A PMP-labelled sugar chain was mixed with 2,5-dihydrobenzoic acid, and examined with MALDI-TOF. The purification and examination method were the same as in Experiment 5.

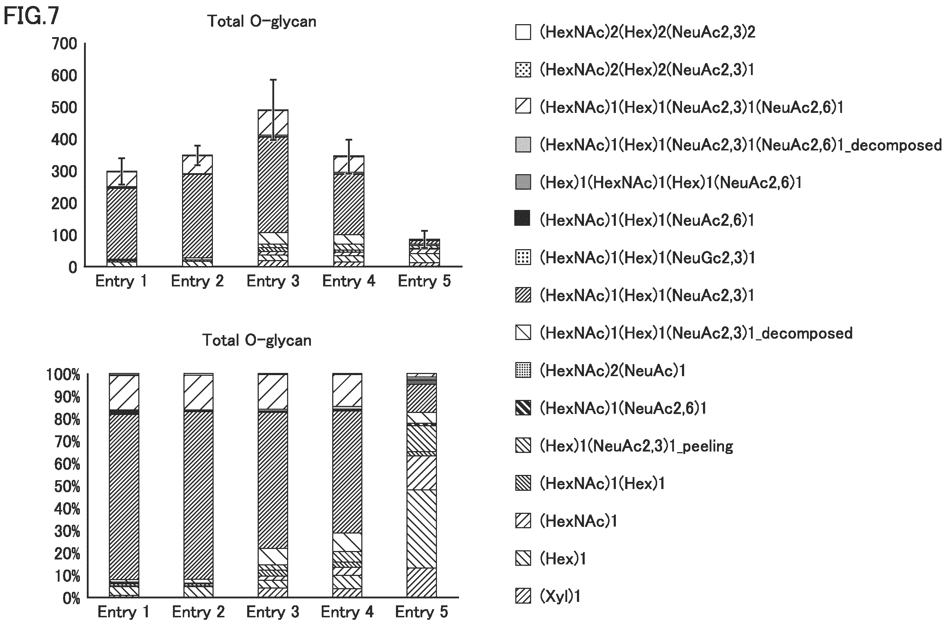

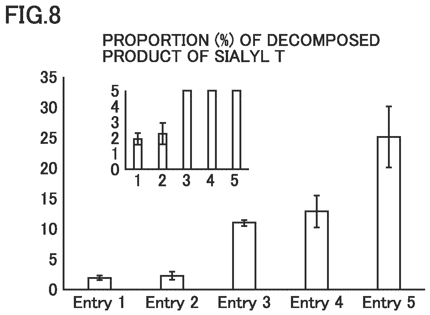

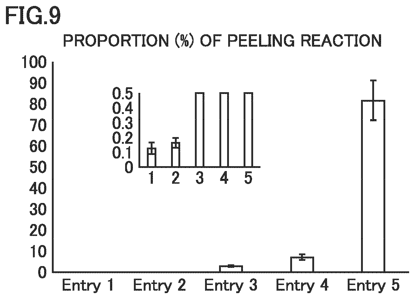

The examination results are shown in Fig. 7 to Fig. 9. The amount of recovery of an O-linked sugar chain was highest in Entry 3 (Fig. 7). On the other hand, the proportion of a decomposed product of sialyl T methylamidated was increased in each of Entries 3 to 5, as compared with Entries 1 and 2 (Fig. 8). The proportion of a decomposed product (peeling reaction) in the β-elimination reaction was increased in each of Entries 3 to 5, as compared with Entries 1 and 2 (Fig. 9).

### [Table 1]

**Table 1 Composition and pH of β-elimination reaction solution**

| | | | | | |
|---|---|---|---|---|---|
| Entry | 1 | 2 | 3 | 4 | 5 |
| PMP (M) | 1 | 1 | 1 | 1 | 1 |
| NaOH (M) | 0.7 | 0.75 | 0.93 | 0.95 | 0.97 |
| pH | 8.7 | 9.1 | 9.9 | 10.1 | 11.9 |

### [Aspects]

A plurality of exemplary embodiments and examples described above are understood by those skilled in the art to be specific examples of the following aspects.

### (Item 1)

A method for analyzing an O-linked sugar chain subjected to linking mode-specific modification of sialic acid contained in a specimen, according to one aspect, includes a modification step of adding a modifier that subjects the O-linked sugar chain to linking mode-specific modification of sialic acid, to a sample including a glycoprotein to which the O-linked sugar chain is linked, a releasing step of releasing the O-linked sugar chain subjected to linking mode-specific modification of sialic acid, from the glycoprotein, and an analysis step of analyzing the released O-linked sugar chain subjected to linking mode-specific modification of sialic acid. According to the method described in Item 1, the linking mode of sialic acid can be discriminated with good accuracy even if a sugar chain contains O-linked sialic acid.

### (Item 2)

In the method described in Item 1, the linking mode-specific modification of sialic acid includes a first reaction that lactonizes sialic acid. The first reaction allows sialic acid to be selectively lactonized depending on the linking mode. In the first reaction, α2,3-sialic acid in a sugar chain is preferably lactonized.

### (Item 3)

In the method described in Item 2, the linking mode-specific modification of sialic acid further includes a second reaction that amidates a lactone structure generated by the first reaction. According to the method described in Item 3, the linking mode-specific modification of sialic acid can be conducted with better accuracy.

### (Item 4)

In the method described in Item 3, the second reaction is conducted with a solution containing at least one selected from the group consisting of ammonia, amine and a salt thereof. According to the method described in Item 4, the linking mode-specific modification of sialic acid can be efficiently conducted.

### (Item 5)

In the method described in Item 3 or Item 4, the second reaction is an aminolysis reaction.

### (Item 6)

In the method described in any of Item 3 to Item 5, sialic acid after the second reaction is modified differently in terms of mass, depending on the linking mode. According to the method described in Item 6, sialic acid modified by linking mode-specific modification can be distinguished by an analysis method such as mass spectrometry.

### (Item 7)

In the method described in Item 6, the linking mode of sialic acid contains α-2,3 sialic acid and α-2,6 sialic acid. According to the method described in Item 7, α-2,3 sialic acid and α-2,6 sialic acid can be distinguished.

### (Item 8)

In the method described in any of Item 2 to Item 7, a solution containing a dehydration-condensation agent is added to the sample to conduct the first reaction. According to the method described in Item 8, different modification depending on the linking mode of sialic acid can be made based on the difference in molecule structure depending on the linking mode of sialic acid.

### (Item 9)

In the method described in any of Item 2 to Item 8, α-2,6 sialic acid is amidated after the first reaction.

### (Item 10)

The method described in any of Item 2 to Item 9 further includes purifying the glycoprotein by a protein precipitation method, after the first reaction. According to the method described in Item 10, the solution used in the upstream step can be removed to enhance the reaction efficiency in the downstream step.

### (Item 11)

In the method described in any of Item 1 to Item 10, the sugar chain is released from the glycoprotein by a β-elimination reaction or a hydrazine decomposition reaction. According to the method described in Item 11, the sugar chain can be chemically released from the glycoprotein.

### (Item 12)

In the method described in Item 11, the β-elimination reaction is conducted at a pH of 11.0 or less. According to the method described in Item 12, an O-linked sugar chain can be efficiently released.

### (Item 13)

The methods described in any of Item 1 to Item 12 further include purifying the sugar chain released, by a solid-phase carrier. According to the method described in Item 13, the released sugar chain can be simply separated from protein.

### (Item 14)

In the method described in any of Item 1 to Item 13, the analysis includes at least one selected from the group consisting of mass spectrometry, chromatography and electrophoresis. According to the method described in Item 14, the linking mode of sialic acid can be distinguished to examine the sugar chain based on the difference in mass generated in a linking mode-specific manner and the influence of separation on chromatography or electrophoresis.

## Claims

1. A method for analyzing an O-linked sugar chain subjected to linking mode-specific modification of sialic acid, the method comprising:
a modification step of adding a modifier that subjects the O-linked sugar chain to linking mode-specific modification of sialic acid, to a sample including a glycoprotein to which the O-linked sugar chain is linked;
a releasing step of releasing the O-linked sugar chain subjected to linking mode-specific modification of the sialic acid, from the glycoprotein; and
an analysis step of analyzing the released O-linked sugar chain subjected to linking mode-specific modification of the sialic acid.

2. The method according to claim 1, wherein the linking mode-specific modification of the sialic acid comprises a first reaction that lactonizes sialic acid.

3. The method according to claim 2, wherein the linking mode-specific modification of the sialic acid further comprises a second reaction that amidates a lactone structure obtained by the first reaction.

4. The method according to claim 3, wherein the second reaction is conducted with a solution containing at least one selected from the group consisting of ammonia, amine and a salt thereof.

5. The method according to claim 3 or 4, wherein the second reaction is an aminolysis reaction.

6. The method according to claim 3 or 4, wherein sialic acid after the second reaction is modified differently in terms of mass, depending on the linking mode.

7. The method according to claim 6, wherein the linking mode of the sialic acid contains α-2,3 sialic acid and α-2,6 sialic acid.

8. The method according to claim 2 or 3, wherein a solution containing a dehydration-condensation agent is added to the sample to conduct the first reaction.

9. The method according to claim 2 or 3, wherein α-2,6 sialic acid is amidated after the first reaction.

10. The method according to claim 2 or 3, further comprising purifying the glycoprotein by a protein precipitation method, after the first reaction.

11. The method according to claim 1 or 2, wherein the sugar chain is released from the glycoprotein by a β-elimination reaction or a hydrazine decomposition reaction.

12. The method according to claim 11, wherein the β-elimination reaction is conducted at a pH of 11.0 or less.

13. The method according to claim 1 or 2, further comprising purifying the sugar chain released, by a solid-phase carrier.

14. The method according to claim 1 or 2, wherein the analysis comprises at least one selected from the group consisting of mass spectrometry, chromatography and electrophoresis.
